# EUROPEAN PATENT SPECIFICATION

(11) **EP 3 030 203 B1**
(45) Date of publication and mention of the grant of the patent: **26.12.2018**
(21) Application number: 14786311.2
(22) Date of filing: 09.09.2014
(51) Int. Cl.: A61F 5/01, A61F 5/37

(54) **BRACE FOR IMMOBILISING A TRAUMATIZED OR OPERATED SHOULDER**
SCHIENE ZUR IMMOBILISIERUNG EINER VERLETZTEN ODER OPERIERTEN SCHULTER
BANDAGE DE DUJARIER POUR IMMOBILISER UNE ÉPAULE TRAUMATISÉE OU OPÉRÉE

(30) Priority: 10.09.2013 IT MI20131491
(43) Date of publication of application: 15.06.2016
(73) Proprietor: Orthoservice AG, 6830 Chiasso (CH)
(72) Inventor: TURCONI, Francesco, 20900 Monza (MB) (IT); ROSSI, Paolo, 6830 Chiasso (CH)
(74) Representative: Branca, Emanuela
(86) International application number: PCT/IB2014/064340
(87) International publication number: WO 2015/036915

(56) References cited:
- EP-A1- 2 591 757
- US-A- 3 404 680
- US-A1- 2003 187 373

## Description

The present invention refers to a brace for immobilising a traumatized or operated shoulder.

The present invention is suitable for being used in the orthopaedic medical field and, in particular, it refers to the field of braces and/or di similar support structures suitable for keeping operated or traumatised body parts in the correct position.

In detail, the object of the present invention focuses on braces intended for immobilizing shoulders of patients who have undergone or have been subjected to: repair of rotator cuffs; subluxations; dislocations; Bankart procedures; repair of soft tissues following traumas; external rotation immobilisation; plastic capsular repair procedures or anterior/posterior stabilisations procedures.

As known, braces intended for immobilising traumatized or operated shoulders, like the ones disclosed and shown in the document US2003/01487373, are usually equipped with a cushion that is placed between the chest and the limb of the patient corresponding to the shoulder involved. The cushion is generally provided with an arm support sling configured to receive the forearm of the respective user. The cushion usually has a series of belts engaged with it that allow the brace to be attached to the body of the user so that the forearm, inserted into the arm support sling and, consequently, the corresponding traumatized or operated shoulder, remain locked in a predetermined position. Generally, the aforementioned braces are provided with an abdominal band configured to envelop and surround the abdominal/lumbar regions of the user to keep the cushion adherent to the body of the same.

Such braces are also provided with a support belt the ends of which engage the cushion from above so as to form a strap the top of which is configured to rest on the contralateral shoulder of the user, i.e. the healthy shoulder not involved in the trauma or operation.

When worn, the belt or support strap of the brace rests on the shoulder opposite the traumatized or operated shoulder and the arm inserted in the arm support sling engaged to the cushion, extending diagonally to the sagittal plane of the user.

The belt or support strap is therefore placed under tension by the arm of the user that tends to pull the arm support sling and the cushion downwards, by gravity. Consequently, the belt or support strap, also pulled downwards by the relaxed arm, exerts a continuous pressure, in an oblique direction, on the base of the user's neck.

Although known braces using a cushion for immobilizing traumatised shoulders allow satisfactory rehabilitation thereof, the Applicant has found that they are not without drawbacks and they can be improved in various aspects, mainly in relation to comfort during use, the bodily well-being of the user, the correct position of the traumatized or operated shoulder, the degree of immobilization thereof, as well as the speed and times needed to rehabilitate the traumatised parts and allow the correct recovery of the kinetic chains involved.

In particular, the Applicant has found that all braces using a cushion for immobilizing traumatised shoulders are provided with belts or support straps that work on the contralateral shoulder generating a series of undesired problems.

Firstly, the diagonal orientation of the belts or support straps, typical of braces using a cushion for immobilizing traumatised shoulders, determines a continuous pressure on the base of the user's neck that, in the long term, begins to cause soreness or even acute pain that can lead to neck pain and/or similar pathologies. In detail, contralateral straps and belts work directly and transversally on the neck/shoulder joint relative to the healthy shoulder significantly stressing both the muscles and the nerve endings in this area. The sensations of discomfort, soreness and pain are rendered more acute and aggravated by the fact that such braces must be worn for particularly long periods, of the order of weeks. Consequently, in order to compensate for unpleasant sensations, as well as the pains that are generated during the use of the aforementioned braces, users can take up incorrect positions and/or postures that lead to further drawbacks. Soreness and pain can even lead to the voluntary removal of the brace with serious consequences for the traumatized or operated shoulder. Among the various incorrect postures and/or positions that users tend to take up during the use of such braces there is the lifting of the traumatized or operated shoulder, with consequent rotation of the scapula: this generates incorrect positioning with respect to the desired immobilization and complicates the rehabilitation procedures and the times needed for them.

The main purpose of the present invention is to provide a brace for immobilising a traumatized or operated shoulder capable of solving the problems encountered in the prior art.

A purpose of the present invention is to provide a brace for immobilising a traumatized or operated shoulder that is comfortable and does not generate sensations of soreness and/or even physical pain.

A further purpose of the present invention is to propose a brace for immobilising a traumatized or operated shoulder that ensures that the correct body position and posture are maintained.

A purpose of the present invention is to provide a brace for immobilising a traumatized or operated shoulder that is capable of simplifying and shortening, in terms of time, the rehabilitation procedures.

The purposes specified above, and yet others, are substantially accomplished by a brace for immobilising a traumatized or operated shoulder as expressed and described in the following claims.

As an example, it will be given the description of a preferred, but not exclusive, embodiment of a brace for immobilising a traumatized or operated shoulder in accordance with the present invention.

The description will be made hereafter with reference to the attached drawings, provided for indicating and therefore not limiting purposes, in which:
figure 1 is a schematic representation in frontal perspective view of a brace for immobilising a traumatized or operated shoulder of a user, in accordance with the present invention, shown while it is worn and adjusted;
figure 2 is a further schematic representation in frontal perspective view of the brace according to figure 1 represented in worn condition;
figure 3 is a schematic representation in frontal view of the brace according to figures 1 and 2 shown in worn condition;
figure 4 is a schematic representation in rear view of the brace according to figures 1 to 3 represented while it is worn and adjusted;
figure 5 is a schematic representation in rear view of the brace according to figures 1 to 4 shown in worn condition.

With reference to the attached figures, reference numeral 1 wholly indicates a brace for immobilising a traumatized or operated shoulder, in particular for shoulders subjected to the repair of rotator cuffs, subluxations, dislocations, Bankart procedures, repair of soft tissues following traumas, external rotation immobilisation, plastic capsular repair or anterior/posterior stabilisations procedures, in accordance with the present invention.

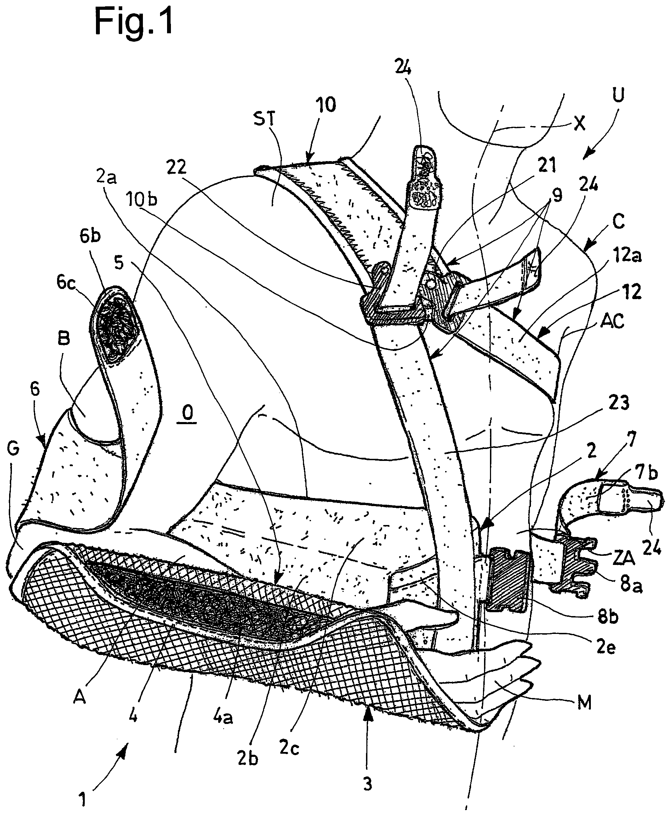

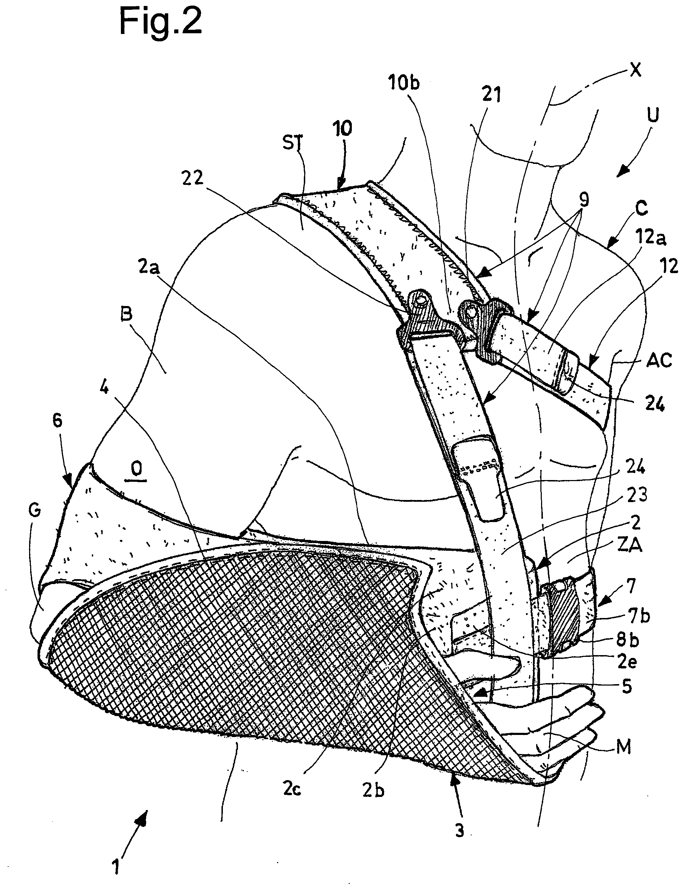

As can be seen in the attached figures, the brace 1 comprises at least one cushion 2 suitable for at least partly adhering to the body C of a user U having a traumatized or operated shoulder ST to be immobilised. In detail, the cushion 2 has a substantially elongated configuration on which it is possible to identify at least one inner surface 2a (figures 1 to 5) configured to remain resting against the body C of the user U and at least one outer surface 2b (figures 1 to 5) facing the opposite side with respect to the inner surface 2a, suitable for receiving, in contact the forearm A (figure 1) of the user U corresponding to the traumatized or operated shoulder ST.

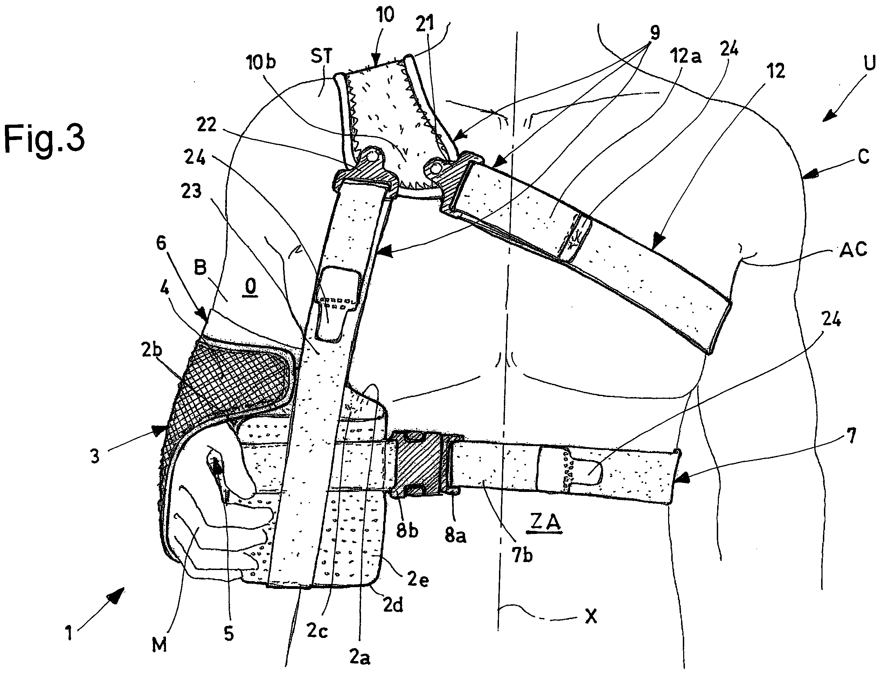

The cushion 2 also has at least one upper surface 2c (figures 1 to 5) and at least one lower surface 2d (figures 3 to 5) that extend between the inner and outer surfaces 2a, 2b.

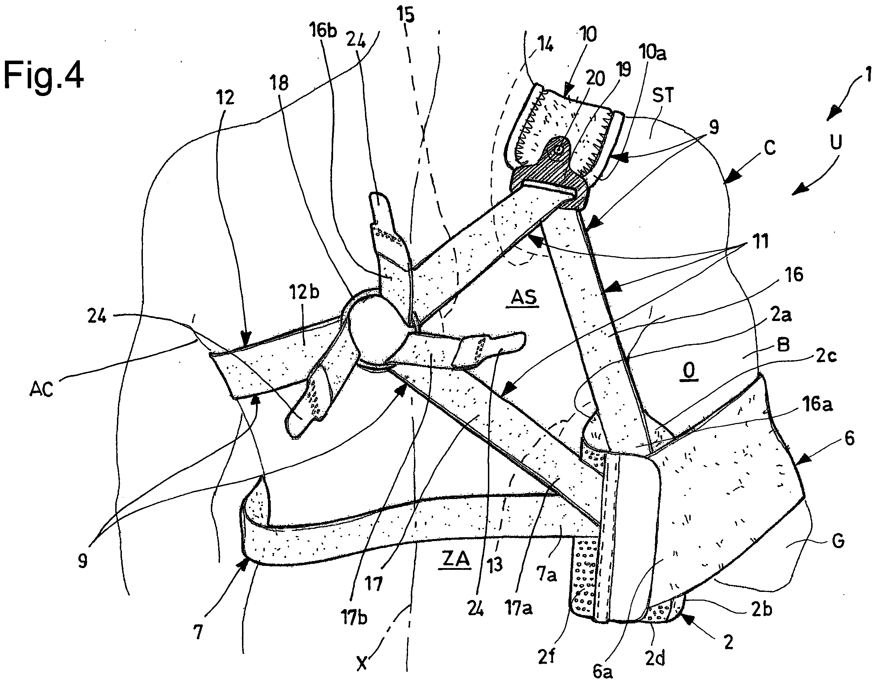

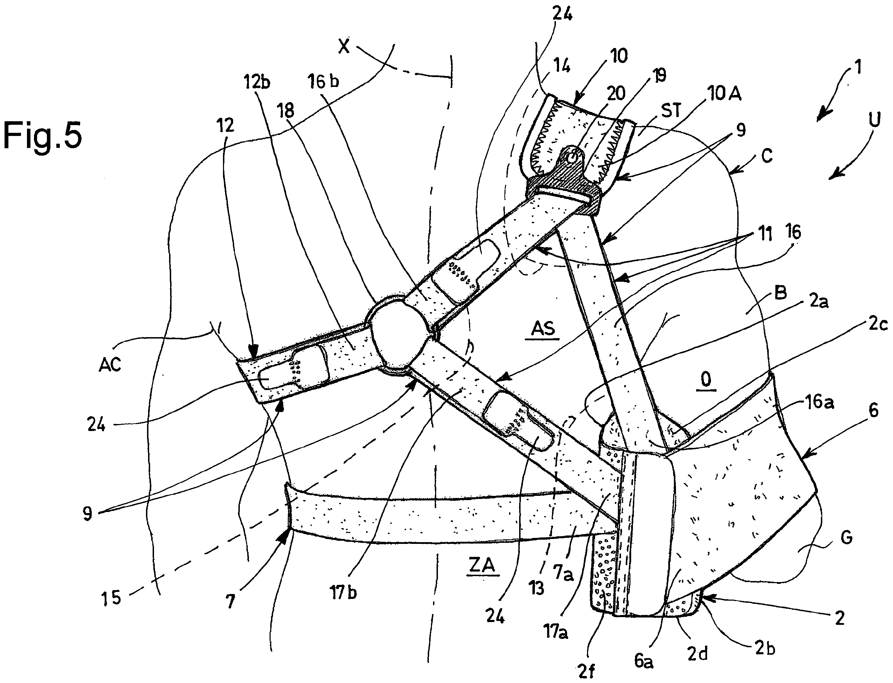

Finally, the cushion 2 has at least one front surface 2e (figures 1 to 3) and at least one rear surface 2f (figures 4 and 5) arranged, the first at the hand M (figures 1 to 3) of the user U corresponding to the traumatized or operated shoulder ST, the second at the elbow G (figures 1, 2, 4 and 5) of the user U corresponding to the traumatized or operated shoulder ST.

The brace 1 also comprises suitable constraining means for the engagement of the forearm A of the user U corresponding to the traumatized or operated shoulder ST to the cushion 2.

Advantageously, the constraining means comprise at least one arm support sling 3 (figures 1 to 3) that extends from the cushion 2, preferably from the outer surface 2b thereof. The arm support sling 3 has at least one free portion 4 (figures 1 to 3) provided with at least one hooking element 4a (figure 1), optionally of the Velcro® type.

It is possible for the portion 4 of the arm support sling 3 to be provided with a plurality of hooking elements 4a distributed so as to permit the correct engagement thereof to the cushion 2.

As can be seen in figures 1 to 3, the free portion 4 of the arm support sling 3 can be engaged to the cushion 2 through the hooking element 4a to delimit along with it at least one compartment 5 for housing the forearm A of the user U corresponding to the traumatized or operated shoulder ST.

In detail, the arm support sling 3 has dimensions and shapes such as to allow the engagement of the hooking element 4a to the upper surface 2c of the cushion 2 so that the compartment 5 for housing the forearm A of the user U, relative to the traumatized or operated shoulder ST, is, at least in part, delimited between the arm support sling 3 and the outer surface 2b of the cushion 2.

The arm support sling 3 described above can also be replaced by one or more belts or bands capable to keeping the forearm A constrained to the cushion 2 according to the best position for the recovery of the traumatized or operated shoulder.

Advantageously, the brace 1 is provided with an auxiliary stabilising band 6 (figures 1 to 5), optionally made of elastic material, for blocking the arm B of the user U relative to the traumatized or operated shoulder ST at the elbow G thereof.

In particular, the stabilising band 6 has a first end 6a (figures 4 and 5) that engages, optionally by stitching (preferably by Velcro®, in order to be able to adjust the length and inclination of the band 6 itself), the cushion 2, preferably the rear surface 2f thereof, and a second end 6b (figure 1), opposite the first end 6a, provided with at least one hooking element 6c, optionally of the Velcro® type, for the engagement of the cushion 2.

Advantageously, the auxiliary stabilising band 6 is extensible astride of the arm B of the user U to at least partially wrap the humerus O thereof and block it against the cushion 2.

Again with reference to the attached figures, the brace 1 comprises at least one abdominal band 7, optionally adjustable length-wise, suitable for enveloping and/or surrounding at least one abdominal and lumbar area ZA of the user U.

The abdominal band 7 is advantageously engaged to the cushion 2, preferably by stitching of a first end 7a thereof (figures 4 and 5) to the rear surface 2f thereof, and it extends from the latter on the opposite side with respect to the arm support sling 3. On the opposite side to the first end 7a, the abdominal band 7 has a second end 7b (figures 1 to 3), optionally provided with a snap blocking device 8a (figures 1 - 3) able to interact with a corresponding blocking device 8b (figures 1 to 3), localized on the cushion 2, preferably at the front surface 2e thereof. Advantageously, the abdominal band 7 is adjustable length-wise to keep the cushion 2 resting against the body C of the user U.

Again with reference to the attached figures, the brace 1 comprises at least one support harness 9 engaged to the cushion 2 to support the latter according to a predetermined position and level.

As can be seen in the attached figures, the support harness 9 has at least one rest portion 10 suitable for engaging at least one shoulder of the user U to support the cushion 2 and the arm support sling 3. Advantageously, the support harness 9 is configured so as to engage, preferably in contact, the traumatized or operated shoulder ST of the user U, in other words the shoulder corresponding to the forearm A that engages the arm support sling 3 and the cushion 2 of the brace 1.

The support harness 9 has, between the cushion 2 and the rest portion 10, at least one triangular structure 11 (figures 4 and 5) suitable for remaining against the body C of the user U at the scapular area AS (figures 4 and 5) of the traumatized or operated shoulder ST thereof.

The support harness 9 also has, between the triangular structure 11 and the rest portion 10, at least one transverse stabilisation belt 12 intended to wrap around the body C of the user U and stabilize the rest portion 10, passing beneath the contralateral axilla cavity AC of the user U on the opposite side to the cushion 2.

Going into detail, as can be seen in figures 4 and 5, the triangular structure 11 has a first vertex 13 arranged substantially at the cushion 2, a second vertex 14 arranged substantially at a rear end 10a of the rest portion 10 of the support harness 9 and a third vertex 15 arranged substantially at the sagittal plane X of the user U.

Each vertex 13, 14, 15 can have a variable width according to the configuration necessary to allow the adaptation of the brace 1 to the body shape of the user U.

Preferably, the triangular structure 11 is made so as to allow the variation of the width of each vertex 13, 14, 15. Of course, the variation of the width of one of the vertices 13, 14, 15 of the triangular structure 11 determines the variation of the width of the other angles 13, 14, 15.

Therefore, the width of the vertices 13, 14, 15 of the triangular structure 11 gives it a reticular configuration capable of providing a barrier to the movement and/or rotation of the scapula relative to the traumatized or operated shoulder ST of the user U.

In accordance with the solution illustrated in figures 4 and 5, the triangular structure 11 of the support harness 9 is provided with at least two belts 16, 17, each of which has a first end 16a, 17a engaged to the cushion 2, preferably to the rear surface 2f of the latter by means of corresponding stitching, and a second end 16b, 17b engaged to a central return element 18, suitably configured to remain in proximity or at the sagittal plane X of the user U.

The first belt 16 of the triangular structure 11, optionally adjustable length-wise, advantageously extends between the cushion 2 and the central return element 18 (figures 4 and 5), preferably through just an upper return element 19, localized on the rear end 10a of the rest portion 10 of the support harness 9. The second belt 17 of the triangular structure 11 also extends between the cushion 2 and the central return element 18 without however engaging any intermediate return element.

As can be seen in figures 4 and 5, the first ends 16a, 17a of the first and second belt 16, 17 define the first vertex 13 of the triangular structure 11, the second ends 16b, 17b of the first and second belt 16, 17 define the third vertex 15 of the triangular structure 11, whereas the first belt 16 defines the second vertex 14 of the triangular structure 11 at the upper return element 19.

Advantageously, the central return element 18 has an annular structure, optionally circular, for the engagement of at least one of the belts 13, 14, 15 of the triangular structure 11 of the support harness 9. The annular structure of the central return element 18 lies on a plane substantially parallel to the body C of the user U during the use of the brace 1 avoiding unpleasant and painful sensations typical of solid bodies sticking into the skin.

According to a further advantageous aspect of the present invention, the upper return element 19 comprises at least one hinging point 20 (figures 4 and 5) to the rear end 10a of the rest portion 10 of the harness 9. In this way, the upper return element 19 is free to rotate, with respect to the rest portion 10, about the hinging point 20 to allow the variations in width of the second vertex 14 of the triangular structure 11.

The upper return element 19 also has at least one slit 20a suitable for receiving - passing through it - a respective belt, preferably the first belt 16, of the triangular structure 11.

As an alternative to the embodiment shown in figures 4 and 5, the triangular structure 11 can also be made through a first belt that extends just between the cushion 2 and the rear end 10a of the rest portion 10 and a second belt that extends between the cushion and the second end 10a of the rest portion 10 through the central return element 18. In this case, the upper return element 19 can be provided with a two-loop structure oriented so as to receive in engagement the corresponding ends of the belts of the triangular structure 11 or two independent bodies, one dedicated to each belt. Unlike the embodiment represented, the second vertex 14 of the triangular structure 11 would, in this case, be defined by the first and by the second belt, whereas the third vertex 15 would be defined just by the second belt at the central return element 18.

It is also possible for the triangular structure 11 to be able to be made through the use of three belts: a first belt extending between the cushion 2 and the rear end 10a of the rest portion 10; a second belt extending between the rear end 10a of the rest portion 10 and the central return element 18; and a third belt extending between the cushion 2 and the central return element 18. Also in this case it is possible to use an upper return element structured so as to have two loops each dedicated to a respective belt or an upper return element that is provided with two independent bodies, each dedicated to a respective belt.

Finally, it is possible for the triangular structure 11 to be made through a single belt having a first end fixed to the cushion 2 and a second end able to be engaged so as to allow length-wise adjustment. In this case, the belt defines, at the return elements 18 and 19, the third and the second vertex 15, 14 of the triangular structure 11 that would be structured so as to allow the belt itself to be crossed and folded over. The aforementioned transverse stabilisation belt 12 of the support harness 9 comprises a first end 12a (figures 1 to 3) engaged to a first front return element 21, which rotatably engages a front end 10b of the rest portion 10 of the support harness itself, and a second end 12b engaged to the central return element 18.

Alongside the first front return element 21 of the support harness 9 is further provided with a second front return element 22, also rotatably engaged to the front end 10b of the rest portion 10. The second front return element 22 supports a front support belt 23, preferably adjustable length-wise, which engages, on the opposite side with respect to the second front return element 22, the cushion 2, in particular the front surface 2e thereof advantageously by means of the corresponding stitching.

As can be seen in figures 1 to 3, the support belt 23 is arranged vertically, in other words substantially parallel to the sagittal plane (X) of the user U, between the rest portion 10 and the cushion 2 so that just the traumatized or operated shoulder ST of the user U can be engaged by the rest portion 10 of the harness 9.

As can be seen in the attached figures, the adjustment of the belts 12, 16, 17, 23 and of the abdominal band 7 is preferably obtainable through the provision of corresponding hooking elements 24, optionally of the Velcro® type, at the free ends thereof. Such hooking elements 24 allow the ends folded around the corresponding return elements 18, 19, 21, 22 along the extension of the corresponding belts 12, 16, 17, 23 to be blocked.

The brace described above solves the problems encountered in the prior art and achieves important advantages.

Firstly, the brace described above allows the optimal immobilization of the traumatised shoulder even containing the movements of the corresponding scapula. The arrangement of the rest portion at the traumatized or operated shoulder allows an increase in comfort of the brace that is consequently reflected in the rehabilitation power thereof, since the user is no longer induced to take up incorrect positions or postures, being able to use the brace for particularly long time periods.

In fact, the elimination of the contralateral rest portion avoids a transversal pressure on the neck of the user that was the cause of soreness and pain. The rest portion localized directly on the traumatized or operated shoulder is connected substantially vertically to the cushion and to the arm support sling, so that the load relative to the arm of the user only bears down on the shoulder thereof, avoiding diagonally involving the base of the neck that is very sensitive to stresses.

Keeping a correct rehabilitation posture and/or position over time allows a considerable reduction in the overall recovery time of the traumatized or operated shoulder also avoiding involving parts of the body not directly affected by the trauma or by the surgical operation.

## Claims

1. Brace (1) for immobilising a traumatized or operated shoulder (ST), in particular for shoulders subjected to the repair of rotator cuffs, subluxations, dislocations, Bankart procedures, repair of soft tissues following traumas, external rotation immobilisation, plastic capsular repair procedures or anterior/posterior stabilisations procedures, said brace (1) comprising:
at least one cushion (2) suitable for at least partly adhering to the body (C) of a user (U) having a traumatized or operated shoulder (ST) to be immobilised;
constraining means for the engagement with a forearm (A) of said user (U), corresponding to said traumatized or operated shoulder (ST), to said cushion (2), said constraining means comprising at least one arm support sling (3) extending from said cushion (2);
at least one abdominal band (7), optionally adjustable, suitable for enveloping and/or surrounding at least one abdominal and lumbar area (ZA) of said user (U), said abdominal band (7) engaging said cushion (2) and extending from the cushion (2) on the opposite side with respect to said constraining means, said abdominal band (7) being suitable for maintaining said cushion (2) against the body (C) of said user (U);
at least one support harness (9) engaging said cushion (2) for supporting the cushion (2) in a predetermined position and level, said support harness (9) having at least one rest portion (10) suitable for engaging at least one shoulder (ST) of said user (U) for supporting said cushion (2) and said constraining means,
said support harness (9) comprising at least one front support belt (23) which during use is arranged vertically, i.e. substantially parallel to a sagittal plane (X) of said user (U), between said rest portion (10) and said cushion (2)
**characterised in that**
the front support belt is arranged such that it - during use - engages and/or rests on the traumatized or operated shoulder (ST) of said user (U), i.e. the shoulder corresponding to the forearm (A) which engages the constraining means and cushion (2) of said brace (1).

2. Brace according to claim 1, wherein said support harness (9) has:
at least one triangular structure (11) suitable for remaining against the body (C) of the user (U) at the scapular area (SC) of the traumatized or operated shoulder (ST) of the same, said triangular structure (11) being localised between said cushion (2) and said rest portion (10) of said support harness (9);
at least one transverse stabilisation belt (12) for surrounding the body (C) of the user (U) on the side opposite to said cushion (2), said stabilisation belt (12) being localised between said triangular structure (11) and said rest portion (10) of said support harness (9).

3. Brace according to claim 2, wherein said triangular structure (11) has:
a first vertex (13) arranged substantially at said cushion (2);
a second vertex (14) arranged substantially at said rear end (10a) of said rest portion (10) of said support harness (9);
a third vertex (15) arranged substantially at the sagittal plane (X) of the user (U), each vertex (13, 14, 15) having a variable width, so as to define a containment net capable of providing a barrier against the movement and/or rotation of the scapula of the user (U) with respect to the traumatized shoulder (ST).

4. Brace (1) according to claim 2 or 3, wherein the triangular structure (11) comprises:
a first belt (16), optionally adjustable length-wise, having a first end (16a) engaged to said cushion (2) and a second end (16b), opposite to the first, engaged to a central return element (18) configured to remain in proximity or at the sagittal plane (X) of the user (U), said first belt (16) extending between said cushion (2) through at least one upper return element (19), preferably only through an upper return element (19), localised on said rest portion (10) of said support harness (9),
a second belt (17), optionally adjustable length-wise, having a first end (17a) engaged to said cushion (2) and a second end (17b), opposite to the first, engaged to said central return element (18), said second belt (17) extending between said cushion (2) and said central return element (18) without crossing the intermediate return elements;
said first ends (16a, 17a) of said first and second belt (16, 17) defining said first vertex (13) of said triangular structure (11), said second ends (16b, 17b) of said first and second belt (16, 17) defining said third vertex (15) of said triangular structure (11), said first belt (16) defining, at said upper return element (19), said second vertex (16) of said triangular structure (11).

5. Brace (1) according to claim 4, wherein said upper return element (19) comprises:
at least one point (20) for hinging to said rest portion (10) of said support harness (9), said upper return element (19) being rotatable with respect to said rest portion (10) at said hinging point (20);
at least one slit (20a) suitable for receiving - crossing it - a respective belt, preferably said first belt (16) of said triangular structure (11).

6. Brace (1) according to claim 4 or 5, wherein said central return element (18) has an annular structure, optionally circular, for the engagement of at least one belt (12, 16, 17), said annular structure of said central return element (18) lying on a plane substantially parallel to the body of said user (U) during the use of said brace (1).

7. Brace (1) according to one or more of claims 4 to 6, wherein said stabilisation transversal belt (12) of said support harness (9) comprises:
a first end (12a) engaged to a first front return element (21) rotatably engaged to a front end (10b) of said rest portion (10) of said support harness (9);
a second end (12b) engaged to said central return element (18) on the side opposite to said triangular structure (11) of said support harness (9).

8. Brace according to any one of the preceding claims, further comprising at least one auxiliary stabilising band (6), optionally made of elastic material, for blocking an arm (B) of said user (U) with respect to said traumatized or operated shoulder (ST) at a relative elbow (G) thereof, said stabilising band (6) having a first end (6a) engaged to said cushion (2) and a second end (6b), opposite to the first end (6a), provided with at least one hooking element (6c) for the engagement thereof to said cushion (2).

9. Brace (1) according to any one of the preceding claims, wherein said means for constraining said forearm (A) of said user (U) corresponding to the traumatized or operated shoulder comprise at least one arm support sling (3), said arm support sling (3) extending from said cushion (2) for delimiting, with the latter, at least one compartment (5) for housing said forearm (A).

## Patentansprüche

1. Schiene (1) zur Immobilisierung einer verletzten oder operierten Schulter (ST), insbesondere für Schultern, die der Wiederherstellung von Rotatorenmanschetten, Subluxationen, Luxationen, Bankart-Operationen, Wiederherstellung von Weichgewebe nach Verletzungen, externen Rotationsimmobilisierung, plastischen Kapselwiederherstellungsverfahren und/oder vorderen/hinteren Stabilisierungsverfahren unterzogen werden, wobei die Schiene (1) Folgendes umfasst:
mindestens ein Polster (2), das geeignet ist, mindestens teilweise am Körper (C) eines Benutzers (U) mit einer zu immobilisierenden verletzten oder operierten Schulter (ST) anzuliegen;
Festlegemittel für die Eingriffsnahme eines Unterarms (A) des Benutzers (U), der der traumatisieren oder operierten Schulter (ST) entspricht, an dem Polster (2), wobei die Festlegemittel mindestens eine Armtrageschlinge (3) umfassen, die sich aus dem Polster (2) erstreckt;
mindestens ein wahlweise einstellbares Bauchband (7), das geeignet ist, mindestens einen Bauch- und Lendenbereich (ZA) des Benutzers (U) zu umhüllen und/oder zu umgeben, wobei das Bauchband (7) das Polster (2) in Eingriff nimmt und sich aus dem Polster (2) auf der gegenüberliegenden Seite in Bezug auf die Festlegemittel erstreckt, wobei das Bauchband (7) dazu geeignet ist, das Polster (2) am Körper (C) des Benutzers (U) zu halten;
mindestens ein Haltegeschirr (9), das das Polster (2) in Eingriff nimmt, um das Polsters (2) in einer vorgegebenen Position und Höhe zu tragen, wobei das Haltegeschirr (9) mindestens einen Auflageabschnitt (10) aufweist, der zur Eingriffsnahme von mindestens einer Schulter (ST) des Benutzers (U) zur Auflage des Polsters (2) und der Festlegemittel geeignet ist,
wobei das Haltegeschirr (9) mindestens einen vorderen Tragriemen (23) umfasst, der während des Gebrauchs senkrecht, d.h.
im Wesentlichen parallel zu einer sagittalen Ebene (X) des Benutzers (U), zwischen dem Auflageabschnitt (10) und dem Polster (2) angeordnet ist,
**dadurch gekennzeichnet, dass**
der vordere Tragriemens derart angeordnet ist, dass er während des Gebrauchs die verletzte oder operierte Schulter (ST) des Benutzers (U) in Eingriff nimmt und/oder darauf liegt, d.h. die Schulter, die dem Unterarm (A) entspricht, der die Festlegemittel und das Polster (2) der Schiene (1) in Eingriff nimmt.

2. Schiene nach Anspruch 1, wobei das Haltegeschirr (9) Folgendes aufweist:
mindestens eine Dreieckstruktur (11), die dazu geeignet ist, am Körper (C) des Benutzers (U) im Bereich des Schulterblatts (SC) der verletzten oder operierten Schulter (ST) desselben anzuliegen, wobei die Dreieckstruktur (11) zwischen dem Polster (2) und dem Auflageabschnitt (10) des Haltegeschirrs (9) angeordnet ist;
mindestens einen Querstabilisierungsgurt (12), um den Körper (C) des Benutzers (U) auf der dem Polster (2) gegenüberliegenden Seite zu umgeben, wobei der Stabilisierungsgurt (12) zwischen der Dreieckstruktur (11) und dem Auflageabschnitt (10) des Haltegeschirrs (9) angeordnet ist.

3. Schiene nach Anspruch 2, wobei die Dreieckstruktur (11) Folgendes aufweist:
einen ersten Scheitelpunkt (13), der im Wesentlichen an dem Polster (2) angeordnet ist;
einen zweiten Scheitelpunkt (14), der im Wesentlichen an dem hinteren Ende (10a) des Auflageabschnitts (10) des Haltegeschirrs (9) angeordnet ist;
einen dritten Scheitelpunkt (15), der im Wesentlichen auf der sagittalen Ebene (X) des Benutzers (U) angeordnet ist, wobei jeder Scheitelpunkt (13, 14, 15) eine variable Breite aufweist, so dass ein Aufnahmenetz definiert wird, das in der Lage ist, eine Barriere gegen die Bewegung und/oder Drehung des Schulterblatts des Benutzers (U) in Bezug auf die verletzte Schulter (ST) bereitzustellen.

4. Schiene (1) nach Anspruch 2 oder 3, wobei die Dreieckstruktur (11) Folgendes umfasst:
einen ersten Gurt (16), der wahlweise in Längsrichtung einstellbar ist, mit einem ersten Ende (16a), das in das Polster (2) eingreift, und einem zweiten Ende (16b), gegenüber dem ersten, das in ein mittleres Rückstellelement (18) eingreift, das ausgestaltet ist, um in der Nähe oder auf der sagittalen Ebene (X) des Benutzers (U) zu bleiben, wobei der erste Gurt (16) sich zwischen dem Polster (2) durch mindestens ein oberes Rückstellelement (19), vorzugsweise nur durch ein oberes Rückstellelement (19) erstreckt, das auf dem Auflageabschnitt (10) des Haltegeschirrs (9) angeordnet ist,
einen zweiten Gurt (17), der wahlweise in Längsrichtung einstellbar ist, mit einem ersten Ende (17a), das in das Polster (2) eingreift, und einem zweiten Ende (17b), gegenüber dem ersten, das in ein mittleres Rückstellelement (18) eingreift, wobei der zweite Gurt (17) sich zwischen den Polster (2) und dem mittleren Rückstellelement (18) erstreckt, ohne die dazwischenliegenden Rückstellelemente zu überkreuzen;
die ersten Enden (16a, 17a) der ersten und zweiten Gurte (16, 17), die den ersten Scheitelpunkt (13) der Dreieckstruktur (11) definieren, wobei die zweiten Enden (16b, 17b) der ersten und zweiten Gurte (16, 17) den dritten Scheitelpunkt (15) der Dreieckstruktur (11) definieren, wobei der erste Gurt (16) an dem oberen Rückstellelement (19) den zweiten Scheitelpunkt (16) der Dreieckstruktur (11) definiert.

5. Schiene (1) nach Anspruch 4, wobei das obere Rückstellelement (19) Folgendes umfasst:
mindestens einen Punkt (20) zum Anlenken an dem Auflageabschnitt (10) des Haltegeschirrs (9), wobei das obere Rückstellelement (19) in Bezug auf den Auflageabschnitt (10) an dem Anlenkpunkt (20) drehbar ist;
mindestens einen Schlitz (20a), der zur Aufnahme eines jeweiligen Gurtes durch Überkreuzen desselben, vorzugsweise des ersten Gurtes (16) der Dreieckstruktur (11) geeignet ist.

6. Schiene (1) nach Anspruch 4 oder 5, wobei das mittlere Rückstellelement (18) eine ringförmige, wahlweise kreisförmige, Struktur für die Eingriffsnahme mindestens eines Gurtes (12, 16, 17) aufweist, wobei die ringförmige Struktur des mittleren Rückstellelements (18) auf einer Ebene liegt, die während des Gebrauchs der Schiene (1) im Wesentlichen parallel zum Körper des Benutzers (U) verläuft.

7. Schiene (1) nach einem oder mehreren der Ansprüche 4 bis 6, wobei der quer verlaufende Stabilisierungsgurt (12) des Haltegeschirrs (9) Folgendes umfasst:
ein erstes Ende (12a), das in ein erstes vorderes Rückstellelement (21) eingreift, das in ein vorderes Ende (10b) des Auflageabschnitts (10) des Haltegeschirrs (9) drehbar eingreift;
ein zweites Ende (12b), das in das mittlere Rückstellelement (18) auf der gegenüberliegenden Seite der Dreieckstruktur (11) des Haltegeschirrs (9) eingreift.

8. Schiene nach einem der vorstehenden Ansprüche, ferner umfassend mindestens ein Hilfsstabilisierungsband (6), das wahlweise aus elastischem Material hergestellt ist, um einen Arm (B) des Benutzers (U) in Bezug auf die verletzte oder operierte Schulter (ST) an einem relativen Ellbogen (G) desselben zu blockieren, wobei das Stabilisierungsband (6) ein erstes Ende (6a) aufweist, das in das Polster (2) eingreift, und ein zweites Ende (6b), das dem ersten Ende (6a) gegenüberliegt und mit mindestens einem Hakenelement (6c) zum Eingreifen desselben in das Polster (2) versehen ist.

9. Schiene (1) nach einem der vorstehenden Ansprüche, wobei die Mittel zum Festlegen des Unterarms (A) des Benutzers (U), der der verletzten oder operierten Schulter entspricht, mindestens eine Armtrageschlinge (3) umfasst, wobei die Armtrageschlinge (3) sich von dem Polster (2) erstreckt, um mit dem letzteren mindestens ein Fach (5) zum Unterbringen des Unterarms (A) zu begrenzen.

## Revendications

1. Orthèse (1) pour immobiliser une épaule traumatisée ou opérée (ST), en particulier pour des épaules soumis à la réparation de coiffes de rotateur, de subluxations, de dislocations, des opérations de Bankart, une réparation de tissus mous à la suite de traumatismes, une immobilisation de rotation externe, des interventions de réparation capsulaire plastique, ou des interventions de stabilisations antérieures/postérieures, ladite orthèse (1) comprenant :
au moins un coussin (2) adapté pour adhérer au moins partiellement au corps (C) d'un utilisateur (U) ayant une épaule traumatisée ou opérée (ST) à immobiliser ;
des moyens de retenue pour l'engagement avec un avant-bras (A) dudit utilisateur (U), correspondant à ladite épaule traumatisée ou opérée (ST), audit coussin (2), lesdits moyens de retenue comprenant au moins une écharpe de support du bras (3) s'étendant à partir dudit coussin (2) ;
au moins une ceinture abdominale (7), éventuellement réglable, adaptée pour envelopper et/ou entourer au moins une zone abdominale et lombaire (ZA) dudit utilisateur (U), ladite ceinture abdominale (7) engageant ledit coussin (2) et s'étendant à partir du coussin (2) sur le côté opposé par rapport auxdits moyens de retenue, ladite ceinture abdominale (7) étant adaptée pour maintenir ledit coussin (2) contre le corps (C) dudit utilisateur (U) ;
au moins un harnais de soutien (9) engageant ledit coussin (2) pour supporter le coussin (2) dans une position et à un niveau prédéterminé, ledit harnais de soutien (9) ayant au moins une portion d'appui (10) adaptée pour engager au moins une épaule (ST) dudit utilisateur (U) pour supporter ledit coussin (2) et lesdits moyens de retenue,
ledit harnais de soutien (9) comprenant au moins une sangle de maintien frontale (23) qui, durant l'utilisation, est agencée verticalement, c'est-à-dire sensiblement parallèle à un plan sagittal (X) dudit utilisateur (U), entre ladite portion d'appui (10) et ledit coussin (2)
**caractérisée en ce que**
la sangle de maintien frontale est agencée de manière que - durant l'utilisation - elle engage et/ou repose sur l'épaule traumatisée ou opérée (ST) dudit utilisateur (U), c'est-à-dire l'épaule correspondant à l'avant-bras (A) qui engage les moyens de retenue et le coussin (2) de ladite orthèse (1).

2. Orthèse selon la revendication 1, dans laquelle ledit harnais de soutien (9) présente :
au moins une structure triangulaire (11) adaptée pour reposer contre le corps (C) de l'utilisateur (U) au niveau de la zone scapulaire (SC) de l'épaule traumatisée ou opérée (ST) de celui-ci, ladite structure triangulaire (11) étant située entre ledit coussin (2) et ladite portion d'appui (10) dudit harnais de soutien (9) ;
au moins une sangle de stabilisation transversale (12) pour entourer le corps (C) de l'utilisateur (U) sur le côté opposé audit coussin (2), ladite sangle de stabilisation (12) étant située entre ladite structure triangulaire (11) et ladite portion d'appui (10) dudit harnais de soutien (9).

3. Orthèse selon la revendication 2, dans laquelle ladite structure triangulaire (11) a :
un premier sommet (13) agencé sensiblement au niveau dudit coussin (2) ;
un deuxième sommet (14) agencé sensiblement au niveau de ladite extrémité arrière (10a) de ladite portion d'appui (10) dudit harnais de soutien (9) ;
un troisième sommet (15) agencé sensiblement au niveau du plan sagittal (X) de l'utilisateur (U), chaque sommet (13, 14, 15) ayant une largeur variable, de manière à définir un filet de confinement capable de fournir une barrière contre le mouvement et/ou la rotation de l'omoplate de l'utilisateur (U) par rapport à l'épaule traumatisée (ST).

4. Orthèse (1) selon la revendication 2 ou 3, dans laquelle la structure triangulaire (11) comprend :
une première sangle (16), éventuellement réglable en longueur, ayant une première extrémité (16a) engagée avec ledit coussin (2) et une deuxième extrémité (16b), opposée à la première, engagée avec un élément de retour central (18) configuré pour rester à proximité ou au niveau du plan sagittal (X) de l'utilisateur (U), ladite première sangle (16) s'étendant entre ledit coussin (2) par le biais d'au moins un élément de retour supérieur (19), de préférence seulement par le biais d'un élément de retour supérieur (19), situé sur ladite portion d'appui (10) dudit harnais de soutien (9),
une deuxième sangle (17), éventuellement réglable en longueur, ayant une première extrémité (17a) engagée avec ledit coussin (2) et une deuxième extrémité (17b), opposée à la première, engagée avec ledit élément de retour central (18), ladite deuxième sangle (17) s'étendant entre ledit coussin (2) et ledit élément de retour central (18) sans croiser les éléments de retour intermédiaires ;
lesdites premières extrémités (16a, 17a) de ladite première et ladite deuxième sangle (16, 17) définissant ledit premier sommet (13) de ladite structure triangulaire (11), lesdites deuxièmes extrémités (16b, 17b) de ladite première et ladite deuxième sangle (16, 17) définissant ledit troisième sommet (15) de ladite structure triangulaire (11), ladite première sangle (16) définissant, au niveau dudit élément de retour supérieur (19), ledit deuxième sommet (16) de ladite structure triangulaire (11).

5. Orthèse (1) selon la revendication 4, dans laquelle ledit élément de retour supérieur (19) comprend :
au moins un point (20) pour l'articulation à ladite portion d'appui (10) dudit harnais de soutien (9), ledit élément de retour supérieur (19) étant rotatif par rapport à ladite portion d'appui (10) au niveau dudit point d'articulation (20) ;
au moins une fente (20a) adaptée pour recevoir - en le croisant - une sangle respective, de préférence ladite première sangle (16) de ladite structure triangulaire (11) .

6. Orthèse (1) selon la revendication 4 ou 5, dans laquelle ledit élément de retour central (18) a une structure annulaire, éventuellement circulaire, pour l'engagement d'au moins une sangle (12, 16, 17), ladite structure annulaire dudit élément de retour central (18) se trouvant dans un plan sensiblement parallèle au corps dudit utilisateur (U) durant l'utilisation de ladite orthèse (1).

7. Orthèse (1) selon une ou plusieurs des revendications 4 à 6, dans laquelle ladite sangle de stabilisation transversale (12) dudit harnais de soutien (9) comprend :
une première extrémité (12a) engagée avec un premier élément de retour frontal (21) engagé de manière rotative avec une extrémité avant (10b) de ladite portion d'appui (10) dudit harnais de soutien (9) ;
une deuxième extrémité (12b) engagée avec ledit élément de retour central (18) sur le côté opposé de ladite structure triangulaire (11) dudit harnais de soutien (9) .

8. Orthèse selon l'une quelconque des revendications précédentes, comprenant en outre au moins une bande de stabilisation auxiliaire (6), éventuellement réalisée en matière élastique, pour bloquer un bras (B) dudit utilisateur (U) par rapport à ladite épaule traumatisée ou opérée (ST) au niveau d'un coude relatif (G) de celui-ci, ladite bande des stabilisation (6) ayant une première extrémité (6a) engagée avec ledit coussin (2) et une deuxième extrémité (6b), opposée à la première extrémité (6a), munie d'au moins un élément d'accrochage (6c) pour l'engagement de celui-ci avec ledit coussin (2).

9. Orthèse (1) selon l'une quelconque des revendications précédentes, dans laquelle lesdits moyens de retenue dudit avant-bras (A) dudit utilisateur (U) correspondant à l'épaule traumatisée ou opérée comprennent au moins une écharpe de support du bras (3), ladite écharpe de support du bras (3) s'étendant à partir dudit coussin (2) pour délimiter, avec ce dernier, au moins un compartiment (5) pour loger ledit avant-bras (A).
